# EUROPÄISCHE PATENTSCHRIFT

(11) **EP 1 210 318 B1**
(45) Veröffentlichungstag und Bekanntmachung des Hinweises auf die Patenterteilung: **05.11.2003**
(21) Anmeldenummer: 00960392.9
(22) Anmeldetag: 04.08.2000
(51) Int. Cl.: C07C 213/08, C07C 213/00, C07B 53/00

(54) **VERFAHREN ZUR HERSTELLUNG VON ADRENALIN**
METHOD FOR THE PRODUCTION OF ADRENALINE
PROCEDE DE FABRICATION D'ADRENALINE

(30) Priorität: 14.08.1999 DE 19938709
(43) Veröffentlichungstag der Anmeldung: 05.06.2002
(73) Patentinhaber: Boehringer Ingelheim Pharma GmbH & Co.KG, 55216 Ingelheim am Rhein (DE)
(72) Erfinder: KLINGER, Franz, Dietrich, 64347 Griesheim (DE); WOLTER, Lienhard, 55606 Hochstetten/Dhaun (DE)
(74) Vertreter: Laudien, Dieter, Dr.
(86) Internationale Anmeldenummer: EP0007573
(87) Internationale Veröffentlichungsnummer: WO01012583

(56) Entgegenhaltungen:
- EP-A- 0 251 164
- EP-A- 0 336 123
- DE-A- 19 902 229
- DE-C- 524 717
- HIDEO TAKEDA ET AL.: "Practical asymmetric synthesis of (R)-(-)-phenylephrine hydrochloride catalysed by (2R,4R)-MCCPM-Rhodium complex" TETRAHEDRON LETTERS., Bd. 30, Nr. 3, 1989, Seiten 367-370, XP002153402 OXFORD GB in der Anmeldung erwähnt
- SAKURABA S ET AL: "EFFICIENT ASYMMETRIC HYDROGENATION OF ALPHA-AMINO KETONE DERIVATIVES A HIGHLY ENANTIOSELECTIVE SYNTHESIS OF PHENYLEPHRINE, LEVAMISOLE, CARNITINE AND PROPRANOLOL" CHEMICAL AND PHARMACEUTICAL BULLETIN,JP,PHARMACEUTICAL SOCIETY OF JAPAN. TOKYO, Bd. 43, Nr. 5, 1. Mai 1995 (1995-05-01), Seiten 738-747, XP000571426 ISSN: 0009-2363 in der Anmeldung erwähnt
- HAYASHI T ET AL: "ASYMMETRIC SYNTHESIS OF 2-AMINO-1-ARYLETHANOLS BY CATALYTIC ASYMMETRIC HYDROGENATION" TETRAHEDRON,NL,ELSEVIER SCIENCE PUBLISHERS, AMSTERDAM, Nr. 5, 1979, Seiten 425-428, XP002028704 ISSN: 0040-4020 in der Anmeldung erwähnt

## Beschreibung

Die vorliegende Erfindung betrifft ein verbessertes Herstellungsverfahren von Adrenalin, bzw. einem Additionssalz davon, in industriellem Maßstab unter Anwendung einer asymmetrischen Hydrierung als Schlüsselschritt und einer speziellen Abfolge von Folgeschritten, wobei als Katalysator [Rh(COD)Cl]₂ und ein chiraler, zweizähniger Phosphinligand wie (2R, 4R)-4-(Dicyclohexylphosphino)-2-(diphenylphosphino-methyl)-N-methyl-aminocarbonyl-pyrrolidin als Katalysatorsystem eingesetzt wird.

### Hintergrund der Erfindung

Adrenalin ist ein zu den Katecholaminen gehörendes Hormon und Neurotransmitter. Im menschlichen Organismus wird es aus Tyrosin gebildet, indem dieses über Dihydroxyphenylalanin, Dopamin und Noradrenalin schließlich zu Adrenalin umgesetzt wird. Adrenalin bewirkt als Sympathikum eine Erregung der adrenergen Rezeptoren des sympathischen Systems mit Steigerung von Pulsfrequenz, Herzminutenvolumen und systolischem Blutdruck, Verminderung der Darmperistaltik, Erschlaffung der Bronchialmuskulatur und Erweiterung der Bronchien, Pupillenerweiterung, Grundumsatzsteigerung durch Förderung des O₂-Verbrauchs, Hyperglykämie und Glukosurie durch Mobilisierung der Glykogenreserven in der Leber sowie u.a. Steigerung der Lipolyse, wodurch die freien Fettsäuren im Blut vermehrt werden. Aufgrund seiner breiten Wirkung ist Adrenalin von hohem kommerziellem Interesse u.a bei der Behandlung des anaphylaktischen Schocks oder als Zusatz zu Lokalanästhetika.
Chemisch ist Adrenalin das L-1-(3',4'-Dihydroxy-phenyl)-2-methylaminoethan-1-ol mit der folgenden Struktur (Formel I):

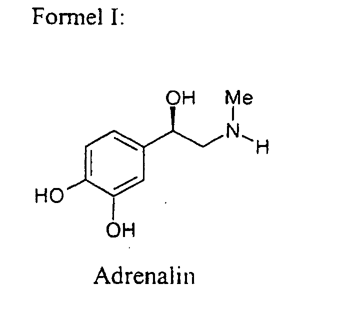

### Stand der Technik

Industriell wird Adrenalin üblicherweise durch nicht stereoselektive Hydrierung von 3',4'-Dihydroxy-2-N-methyl-amino-acetophenon oder einem Derivat davon mit geschützten OH-Funktionen oder Aminofunktion und anschließender Racematspaltung hergestellt.

Enantioselektive Synthesen sind ebenfalls bekannt. Eine davon wird beispielsweise in Tetrahedron Letters 5 (1979), 425 - 428 beschrieben. Danach wird 3',4'-Dihydroxy-2-N-methyl-amino-acetophenon unter Katalyse eines chiralen Hydroxyalkylferrocenylphosphins durch Hydrierung unter einem Wasserstoffdruck von ca. 50 x 10⁵Pa zu Adrenalin umgesetzt. Die Menge des Katalysators zum Substrat liegt bei ca. 1:100 bezogen auf das molare Verhältnis. Unter diesen Bedingungen findet man nach ca. 2 bis 4 Tagen Reaktionszeit R-1-(3',4'-Dihydroxyphenyl)-2-methylamino-ethan-1-ol (Adrenalin) in einem Enantiomerenüberschuß gegenüber dem S-Enantiomeren von 60 % ee.
Zur Herstellung von Adrenalin in industriellem Maßstab ist jedoch dieses Verfahren aufgrund mehrerer Nachteile nicht geeignet: Das Produkt ist trotz der Verwendung großer Mengen an Katalysator im asymmetrischen Reaktionsschritt ohne aufwendige Reinigungsprozeduren für pharmazeutische Zwecke nicht ausreichend rein herstellbar, da Adrenalin durch diese Reaktion nur als Mischung zusammen mit einem relativ hohen Anteil des Antipoden als Verunreinigung zugänglich ist.
Die relativ lange Reaktionszeit des asymmetrischen Hydrierungsschritts von
2 bis 4 Tagen stellt zudem gerade für industrielle Zwecke einen apperativ sehr aufwendigen und teueren Reaktionsschritt mit einem nicht zu vernachlässigenden Sicherheitsrisko dar.

Achiwa et al. beschreiben in Tetrahedron Letters 30 (1989), 367 - 370 und Chem. Pharm. Bull. 43 (5) (1995) 738 - 747 einen asymmetrischen Rhodium-Katalysator, der für die Herstellung von L-Phenylephrin eingesetzt wurde. Mittels asymmetrischer Hydrierung wird innerhalb von 20 Stunden 3'-Benzyloxy-2-(N-benzyl-N-methyl)-aminoacetophenonhydrochlorid mit Wasserstoff in Gegenwart von [Rh(COD)Cl]₂/(2R, 4R)-4-(Dicyclohexylphosphino)-2-(diphenylphosphino-methyl)-N-methyl-aminopyrrolidin als Katalysator reduziert. Nach Filtration, Einengung des Reaktionsgemischs und Abspaltung der benzylischen Stickstoff-Schutzgruppe wird Phenylephrin als Produkt erhalten. Dabei fällt neben dem L-Enantiomeren das D-Enantiomere mit einem Anteil von mindestens 7,5% als Verunreinigung an (85% ee). Der genaue Mechanismus der Rhodium-katalysierten asymmetrischen Hydrierung ist bisher nicht bekannt.

Der Nachteil dieses Verfahrens besteht im wesentlichen darin, daß das erhaltene L-Phenylephrin nicht in wirtschaftlicher Weise im industriellen Maßstab auf eine für die Verwendung als Arzneimittel erforderliche Reinheit aufgereinigt werden kann. Darüberhinaus dauert die Hydrierungsreaktion mit über 20 Stunden relativ lange, was mit den bereits oben geschilderten Nachteilen verbunden ist.

Die Herstellung von Adrenalin nach diesem Verfahren ist nicht bekannt.

### Beschreibung der Erfindung

Die vorliegende Erfindung betrifft ein neues Herstellungsverfahren für Adrenalin durch asymmetrische Hydrierung unter Überwindungen der aus dem Stand der Technik bekannten bzw. der oben aufgeführten Schwierigkeiten und Nachteile.

Eines der wesentlichen Ziele der vorliegenden Erfindung ist es, ein Verfahren zu entwickeln, durch das Adrenalin in hoher optischer und chemischer Reinheit hergestellt werden kann. Damit soll z.B. die Gefahr einer Verunreinigung von Arzneistoffen, die Adrenalin als Wirkstoff enthalten, mit dem unerwünschten Antipoden minimiert werden.

Ein weiteres Ziel der Erfindung besteht darin, ein Verfahren zu entwickeln, durch das weitgehend enantiomerenreines Adrenalin in einfacher Weise, d.h. ohne aufwendige Reinigungsschritte, hergestellt werden kann.

Ein weiteres Ziel der Erfindung besteht darin, Adrenalin über ein stereoselektives Verfahren herzustellen, um Reaktionsschritte zu vermeiden, bei denen chirale Zwischenverbindungen bzw. das chirale Endprodukt Adrenalin als Racemat zusammen mit seinem Antipoden in einer ähnlichen Menge anfällt.

Ein weiteres Ziel des erfindungsgemäßen Verfahrens ist es, die für die Herstellung von Adrenalin notwendigen Hydrierungszeiten möglichst gering zu halten, um die u.a. mit der Verwendung von unter hohem Druck stehendem Wasserstoff verbundenen Kosten und Gefahren zu reduzieren.

Ein weiteres Ziel der vorliegenden Erfindung ist es, dem Fachmann ein Herstellverfahren für Adrenalin an die Hand zu geben, das diesen in großen Mengen benötigten Stoff ausgehend von einfach zugänglichen Edukten auf kostengünstige Weise zugänglich macht.

Überraschenderweise wurde nun gefunden, daß Adrenalin bzw. dessen Sulfat in außergewöhnlich hoher optischer Reinheit aus 3'.4'-Dihydroxy-2-N-benzyl-N-methyl-aminoacetophenon **1** unter Anwendung einer asymmetrischen Hydrierung mit [Rh(COD)Cl]₂/(2R, 4R)-4-Dicyclohexylphosphino)-2-(diphenylphosphino-methyl)-N-methylaminocarbonylpyrrolidin (MCCPM) als Katalysatorsystem und einer speziellen Abfolge von Folgeschritten zugänglich ist. Die in der Summenformel benutzte Abkürzung COD steht für Cyclooctadien.

### Beschreibung der Erfindung im Detail

Bei einem Mol-Verhältnis Katalysator zu Substrat von ca. 1:1500 (siehe Beispiel) kann man durch das erfindungsgemäße Verfahren ausgehend von Benzyladrenalon (3',4'-Dihydroxy-2-N-benzyl-N-methyl-amino-acetophenon) **1** Adrenalinsulfat **3** in einer optischen Reinheit von 98% ee und mehr (HPLC) erhalten (Reaktionsschema 1). 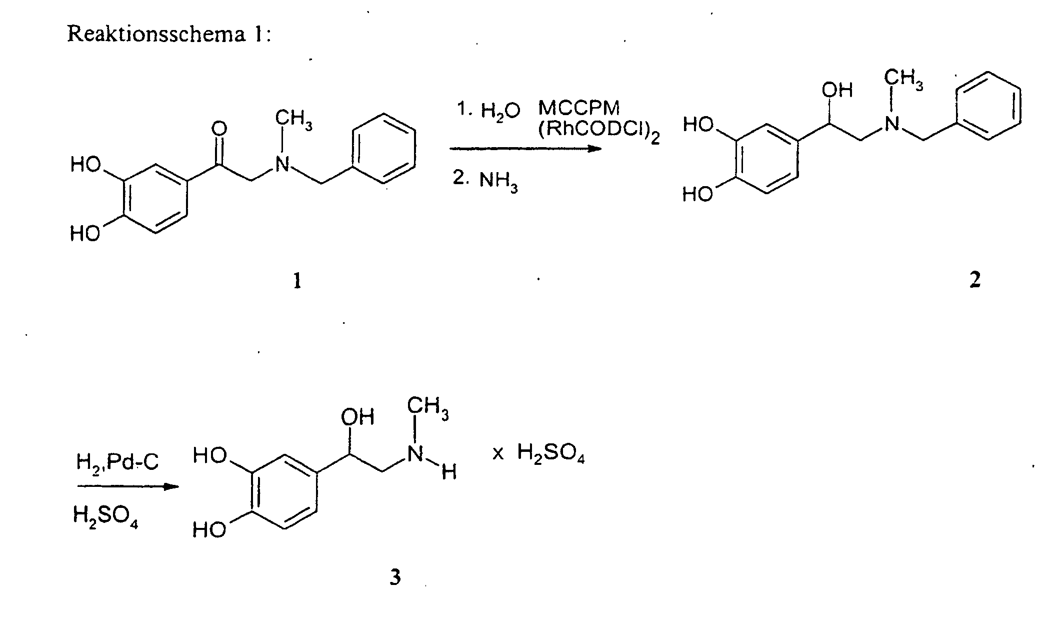 Gemäß Reaktionsschema 1 wird zunächst 3',4'-Dihydroxy-2-N-benzyl-N-methyl-aminoacetophenon **1** durch asymmetrische Hydrierung unter Katalyse von [Rh(COD)Cl]₂/(2R, 4R)-4-(Dicyclohexylphosphino)-2-(diphenylphosphino-methyl)-N-methyl-aminopyrrolidin zur optisch aktivem Benzyladrenalinbase (R-1-(3',4'-dihydroxyphenyl)-2-N-Benzyl-N-methyl-amino-ethan-1-ol) **2** umgesetzt (Reaktionsschritt 1). Diese wird anschließend im Basischen durch Zugabe von Ammoniak gefällt (Reaktionsschritt 2). In einem 3. Reaktionsschritt wird dann die benzylische Schutzgruppe durch Hydrieren mit Wasserstoff und Palladium, bevorzugt Palladium auf Kohle, in schwefelsaurer Lösung entfernt, so daß das Adrenalinsulfat **3** entsteht.

Für die einfache Herstellung des fast optisch reinen Adrenalins bzw. dessen Sulfats **3** stellt neben der asymmetrischen Hydrierung mit dem oben beschriebenen Rhodium-Katalysator die Fällung des N-Benzyladrenalins **2** einen wichtigen Schritt dar. Durch diese beiden Schritte zusammen, asymmetrische Hydrierung plus Fällen des Benzyladrenalins im Basischen, wird in hoher optischer Reinheit in einfacher Weise eine Zwischenverbindung gewonnen, aus der sich in einem weiteren einfachen Reaktionsschritt Adrenalin bzw. dessen Säureadditionssalz in hoher optischer Reinheit gewinnen läßt.

Als Edukt 1 kann neben 3',4'-Dihydroxy-2-N-benzyl-N-methyl-amino-acetophenon ein anderes Derivat von 3',4'-Dihydroxy-2-N-methyl-amino-acetophenon (Adrenalon) eingesetzt werden, bei dem die Stickstofffunktion entweder nicht weiter geschützt ist, als Salz geschützt ist oder mit einer anderen Schutzgruppe als der Benzylschutzgruppe geschützt ist. Für eine solche Schutzgruppe eignet sich z.B. die tert-Butylcarbonyl-, die 9-Fluorenylmethylcarbonyl -oder eine andere aus dem Stand der Technik einschlägig bekannte Stickstoffschutzgruppe. Bevorzugt sind N-geschützte 1-(3',4'-Dihydroxy)-2- N-methyl-amino-acetophenon-Derivate mit einer Schutzgruppe, die unter den Reaktionsbedingungen des ersten Reaktionsschritts (asymmetrische Hydrierung) stabil ist. Als Edukt wird besonders bevorzugt 3',4'-Dihydroxy-2-N-benzyl-N-methyl-amino-acetophenon **1** eingesetzt. Das Edukt **1** kann sowohl als freie Base oder als Salz, z.B. mit ener anorganischen Säure, vorliegen.

Als Katalysator wird erfindungsgemäß [Rh(COD)Cl]₂ und ein chiraler, zweizähniger Phosphinligand eingesetzt. Bevorzugt wird (2R, 4R)-4-(Dicyclohexylphosphino)-2-(diphenylphosphino-methyl)-N-methyl-aminocarbonylpyrrolidin (RR-MCCPM) als Katalysator eingesetzt.

Die Herstellung dieses Katalysators ist aus dem Stand der Technik bekannt [EP-A-0 251 164, EP-A-O 336 123]. Erfindungsgemäß kann der Katalysator auch polymergebunden vorliegen, z.B. indem der chirale Ligand (2R, 4R)-4-Dicyclohexylphosphino)-2-(diphenylphosphinomethyl)-N-methyl-aminocarbonyl) pyrrolidin z.B. über die Phenylgruppen an ein Polymer gebunden ist. Dabei schließt die Verwendung solcher polymergebundener Liganden den gleichzeitigen Einsatz von nicht-polymergebundenem Liganden nicht zwingend aus. Solche polymergebunden Katalysatoren sind insbesondere für eine einfache Reinigung des Produktes von Vorteil.

Der Katalysator wird entweder als vorgefertigte, sauerstofffreie Lösung von [Rh(COD)Cl]₂ und Ligand eingesetzt oder *in situ* aus [Rh(COD)Cl]₂ und Ligand in Gegenwart des 3',4'-Dihydroxy-2-N-benzyl-N-methyl-amino-acetophenon **1** sauerstofffrei unter Schutzgasatmosphäre oder Wasserstoffatmosphäre hergestellt.

Das Mol-Verhältnis von Edukt **1** zu Katalysator liegt im erfindungsgemäßen Verfahren zwischen 500:1 und 10000:1, bevorzugt zwischen 500:1 und 3000:1, insbesondere bevorzugt zwischen 1000:1 und 2000:1 und besonders bevorzugt bei ca. 1500:1.

Als Reaktionsmedium für den ersten Reaktionsschritt (asymmetrische Hydrierung mit dem Rhodiumkatlaysator) dient ein protisches Lösungsmittel, welches bevorzugt vor Verwendung entgast wird. Bevorzugt ist ein C₁ bis C₃-Alkohl, namentlich Methanol, Ethanol, Propanol oder iso-Propanol, bevorzugt Methanol oder Ethanol, insbesondere bevorzugt Methanol. Gegebenenfalls kann das Lösungsmittel Wasser enthalten.

Die Reaktionstemperatur dieses ersten Schrittes liegt bevorzugt zwischen 40 und 70°C, besonders bevorzugt bei 45 bis 55°C.

Der Wasserstoffdruck beträgt 10 bis 100 x 10⁵Pa, bevorzugt 10 bis 50 x 10⁵Pa und insbesondere bevorzugt 15 bis 25 x 10⁵Pa.

Dieser erste Reaktionsschritt ist nach 2 bis 8 Stunden, bevorzugt 4 bis 6 Stunden beendet.

Anschließend wird das Lösungsmittel durch Destillation stark eingeengt, gegebenenfalls mit Wasser verdünnt und mit Aktivkohle versetzt. Nachdem die Aktivkohle wieder abfiltriert worden ist, wird der Reaktionsansatz mit Wasser und bevorzugt dem gleichen Lösungsmittel, das für die asymmetrische Hydrierung eingesetzt wurde, verdünnt und mit einer Base versetzt, um das N-Benzyladrenalin (L-1-(3',4'-Dihydroxy-phenyl)-2-N-benzyl-N-methyl-amino-ethan-1-ol) **2** in hohen optischen Ausbeuten auszufällen.

Als Base eignen sich schwache organische oder anorganische Basen. In beiden Fällen sind besonders Stickstoffbasen bevorzugt. Bei den organischen Basen eignen sich besonders Stickstoffbasen wie Pyridin, Piperidin, Triethylamin, Diethylamin, Ethyl-isopropylamin, Methylamin oder Derivate davon, sofern sie in dem Lösungsmittel löslich sind. Unter den anorganischen Basen ist besonders Ammoniak bevorzugt.
Besonders bevorzugt ist Ammoniak.

Das so erhaltene weitgehend enantiomerenreine N-Benzyladrenalin **2** wird in einem dritten Schritt mit Wasserstoff hydriert. Bevorzugt wird dabei ein Palladiumkatalysator, besonders bevorzugt Palladium auf Kohle eingesetzt. Bevorzugt findet diese Hydrierung im Sauren statt. Dabei wird der pH-Wert der Lösung durch Zugabe von Säure auf 4 bis 6, besonders bevorzugt auf 5 bis 6 eingestellt.

Das Lösungsmittel für diesen Reaktionsschritt ist Wasser, ein C₁ bis C₃-Alkohl, namentlich Methanol, Ethanol, Propanol oder iso-Propanol oder ein Gemisch daraus. Bevorzugt sind Wasser, Wasser-Methanol-Gemische oder Methanol. Besonders bevorzugt ist Wasser.

Zum Ansäuern der Lösung können anorganische oder organische Säuren benutz werden. Als organische Säuren seien beispielhaft genannt: Ameisensäure, Essigsäure, Propansäure, Weinsäure, Äpfelsäure, Bernsteinsäure, Zitronensäure. Als anorganische Säuren seien beispielhaft genannt: Schwefelsäure, Salzsäure, Phosphorsäure. Bevorzugt ist Schwefelsäure.

Die Reaktionstemperatur für diesen Reaktionsschritt beträgt zwischen 40 und 80°C, bevorzugt zwischen 50 und 70°C und besonders bevorzugt beträgt sie 60°C.

Der Wasserstoffdruck beträgt 1 bis 5 x 10⁵Pa, bevorzugt 2 bis 3 x 10⁵Pa.

Durch das erfindungsgemäße Verfahren ist Adrenalin über alle drei Reaktionsschritte in einer Gesamtausbeuten von 75% und mehr bei einer optischen Reinheit von 98% ee und mehr und einer chemischen Reinheit von 98% und mehr zugänglich.

Der Vorteil des erfindungsgemäßen Verfahrens liegt darin, daß gegenüber den aus dem Stand der Technik vergleichbaren Verfahren die Menge an Katalysator deutlich verringert werden kann bzw. die Reaktionszeit der asymmetrischen Hydrierung deutlich reduziert werden kann bei gleichzeitiger Erhöhung der optischen Ausbeute.

Daneben ermöglicht das erfindungsgemäße Verfahren eine optische Aufreinigung bereits auf der Stufe des Zwischenprodukts N-Benzyladrenalin **2** und damit die einfache Gewinnung von Adrenalin in hoher optischer Reinheit.

Das erfindungsgemäße Verfahren soll nun durch das nachfolgende Beispiel erläutert werden. Dieses Beispiel dient nur zur Veranschaulichung und ist nicht als limitierend anzusehen.

### Beispiel

### Herstellung der Katalysator-Lösung:

In 10 ml entgastes Methanol werden unter Schutzgas 6 mg Dichloro-bis-[cycloocta-1,5-dien)rhodium (I)] und 8,2 mg RR-MCCPM (2R,4R)-4-(Dicyclohexyl-phosphino)-2-(diphenylphosphino-methyl)- N-methyl-aminocarbonylpyrrolidin) eingetragen und 30 min. bei Raumtemperatur gerührt.

### Herstellung von Adrenalin

7,4 g des Hydrochlorids des Benzyladrenalons **1** werden in ca. 60 ml Methanol (entgast) gelöst, mit 0,07 ml Triethylamin sowie 10 ml der Katalysatorlösung (entsprechend 6 mg (RhCODCl)₂ und 8,2 mg RR-MCCPM) versetzt und bei ca. 50°C und ca. 20 x 10⁵Pa Wasserstoffdruck hydriert. Nach beendeter Reaktion wird das Methanol größtenteils abdestilliert, mit ca. 30 ml Wasser und ca. 0,5 g Aktivkohle versetzt, 30 min gerührt und abfiltriert. Anschließend wird mit ca. 10 ml Wasser und ca. 15 ml Methanol und durch Zugabe von ca. 4 ml Ammoniak (ca. 25 %ig) N-Benzyladrenalin **2** ausgefällt und abfiltriert. (R.T.)
Ausbeute 6 g = 90 %.

Benzyladrenalin **2** wird in ca. 10 ml Wasser und ca. 5 ml 18 %iger Schwefelsäure gelöst (pH: ca. 5,5), mit ca. 50 mg Palladium-Kohle (10 %ig) versetzt und bei ca. 60 °C und 2 10⁵Pa Wasserstoffdruck hydriert. Anschließend wird auf ca. das halbe Volumen eingeengt, mit ca. 20 ml Methanol versetzt und abgekühlt. Das kristalline Produkt (Adrenalinsulfat **3**) wird abfiltriert und getrocknet.

Ausbeute über alle Stufen zusammen: ca. 4,5 g (ca. 75 %),
opt. Reinheit: > 98 % ee (HPLC)
chem. Reinheit: > 98 % (HPLC).

## Patentansprüche

1. Herstellungsverfahren von Adrenalin oder eines Adrenalinadditionssalzes, **dadurch gekennzeichnet, daß** man in einem ersten Schritt als Edukt N-geschütztes Adrenalon einer asymmetrischer Hydrierung mit [Rh(COD)Cl]₂ und einem chiralen, zweizähnigen Phosphinliganden als Katalysatorsystem unterwirft, danach in einem zweiten Schritt das entstandene N-geschützte Adrenalin im Basischen ausfällt und in einem dritten Schritt die N-Schutzgruppe im Sauren abspaltet.

2. Verfahren nach Anspruch 1, **dadurch gekennzeichnet daß** im ersten Schritt als Edukt N-Benzyl-adrenalon **1** eingesetzt wird, das im ersten Reaktionsschritt zu N-Benzyladrenalin **2** umgesetzt wird, das im zweiten Schritt gefällt wird und im dritten Schritt durch Abspaltung der benzylischen Stickstoffschutzgruppe durch Hydrierung in Gegenwart eines Palladium-Katalysators, bevorzugt Palladium auf Kohle, zu Adrenalin oder dessen Säureadditionssalz umgesetzt wird.

3. Verfahren nach Anspruch 1 oder 2, **dadurch gekennzeichnet daß** der Phosphinligand (2R, 4R)-4-(Dicyclohexylphosphino)-2-(diphenylphosphino-methyl)-N-methylaminocarbonyl-pyrrolidin ist.

4. Verfahren nach Anspruch 1 oder 2, **dadurch gekennzeichnet daß** der Phosphinligand polymergebundenes (2R, 4R)-4-(Dicyclohexylphosphino)-2-(diphenylphosphinomethyl)-N-methyl-aminocarbonyl-pyrrolidin ist.

5. Verfahren nach einem der Ansprüche 1 bis 4, **dadurch gekennzeichnet, daß** die asymmetrische Hydrierung in einem Temperaturbereich von 40°C bis 70°C, bevorzugt 45°C bis 55°C durchgeführt wird.

6. Verfahren nach einem der vorangegangenen Ansprüche 1 bis 5, **dadurch gekennzeichnet, daß** die asymmetrische Hydrierung unter einem Druck von 10 x 10⁵Pa bis 100 x 10⁵Pa, bevorzugt 10 bis 50 x 10⁵Pa durchgeführt wird.

7. Verfahren nach Anspruch 6, **dadurch gekennzeichnet, daß** die asymmetrische Hydrierung unter einem Druck von 15 bis 25 x 10⁵Pa durchgeführt wird.

8. Verfahren nach einem der Ansprüche 1 bis 7, **dadurch gekennzeichnet, daß** die asymmetrische Hydrierung in einem protischen Lösungsmittel durchgeführt wird.

9. Verfahren nach Anspruch 8, **dadurch gekennzeichnet, daß** die asymmetrische Hydrierung in Methanol, Ethanol, Propanol oder iso-Propanol als Lösungsmittel durchgeführt wird.

10. Verfahren nach Anspruch 9, **dadurch gekennzeichnet, daß** die asymmetrische Hydrierung in Methanol als Lösungsmittel durchgeführt wird.

11. Verfahren nach einem der vorangegangenen Ansprüche 9 oder 10, **dadurch gekennzeichnet, daß** das Lösungsmittel für die asymmetrische Hydrierung Wasser enthält.

12. Verfahren nach einem der vorangegangenen Ansprüche 1 bis 11. **dadurch gekennzeichnet, daß** das Mol-Verhältnis Edukt zum Rhodiumkatalysator in der asymmetrischen Hydrierung zwischen 500:1 und 10000:1, bevorzugt zwischen 500:1 und 3000:1 beträgt.

13. Verfahren nach Anspruch 12, **dadurch gekennzeichnet, daß** das Mol-Verhältnis Edukt zum Rhodiumkatalysator in der asymmetrischen Hydrierung zwischen 1000:1 und 2000:1 beträgt.

14. Verfahren nach einem der vorangegangenen Ansprüche 1 bis 13, **dadurch gekennzeichnet, daß** der Rhodiumkatalysator für die asymmetrische Hydrierung als vorgefertigte Lösung eingesetzt wird.

15. Verfahren nach einem der vorangegangenen Ansprüche 1 bis 13, **dadurch gekennzeichnet, daß** der Rhodiumkatalysator für die asymmetrische Hydrierung *in situ* erzeugt wird.

16. Verfahren nach einem der vorangegangenen Ansprüche 1 bis 14, **dadurch gekennzeichnet, daß** die Reaktionszeit für die asymmetrische Hydrierung zwischen 2 und 8 Stunden, bevorzugt zwischen 4 und 6 Stunden liegt.

17. Verfahren nach einem der vorangegangenen Ansprüche 1 bis 16, **dadurch gekennzeichnet, daß** im zweiten Reaktionsschritt als Base eine Stickstoffbase, bevorzugt Ammoniak zum Fällen des N-geschützten Adrenalins verwendet wird.

18. Verfahren nach einem der vorangegangenen Ansprüche 1 bis 17, **dadurch gekennzeichnet, daß** im dritten Reaktionsschritt Schwefelsäure, Salzsäure oder Phosphorsäure, bevorzugt Schwefelsäure zum Ansäuern des Lösungsmittels verwendet wird.

19. Verfahren nach einem der vorangegangenen Ansprüche 1 bis 18, **dadurch gekennzeichnet, daß** der pH-Wert des dritten Schritts zwischen 5 und 6 liegt.

20. Verfahren nach einem der vorangegangenen Ansprüche 1 bis 19, **dadurch gekennzeichnet, daß** die Reaktionstemperatur des dritten Reaktionsschritts 40 bis 80°C, bevorzugt 50 bis 70°C und besonders bevorzugt 60°C beträgt.

## Claims

1. Process for preparing adrenaline or an addition salt of adrenaline, **characterised in that** in a first step N-protected adrenalone as the educt is subjected to asymmetric hydrogenation with [Rh(COD)Cl]₂ and a chiral, bidentate phosphine ligand as catalyst system, then in a second step the N-protected adrenaline produced is precipitated in the basic range and in a third step the N protecting group is cleaved in the acid range.

2. Process according to claim 1, **characterised in that** in the first step N-benzyl-adrenalone **1** is used as educt, which is reacted in the first reaction step to form N-benzyladrenaline **2**, which is precipitated in the second step and is reacted in the third step by cleaving the benzyl nitrogen protecting group by hydrogenation in the presence of a palladium catalyst, preferably palladium on charcoal, to obtain adrenaline or an acid addition salt thereof.

3. Process according to claim 1 or 2, **characterised in that** the phosphine ligand is (2R, 4R)-4-(dicyclohexylphosphino)-2-(diphenylphosphino-methyl)-N-methyl-aminocarbonyl-pyrrolidine.

4. Process according to claim 1 or 2, **characterised in that** the phosphine ligand is polymer-bound (2R, 4R)-4-(dicyclohexylphosphino)-2-(diphenylphosphino-methyl)-N-methyl-aminocarbonyl-pyrrolidine.

5. Process according to one of claims 1 to 4, **characterised in that** the asymmetric hydrogenation is carried out in a temperature range from 40°C to 70°C, preferably 45°C to 55°C.

6. Process according to one of the preceding claims 1 to 5, **characterised in that** the asymmetric hydrogenation is carried out under a pressure of 10 x 10⁵ Pa to 100 x 10⁵ Pa, preferably 10 to 50 x 10⁵ Pa.

7. Process according to claim 6, **characterised in that** the asymmetric hydrogenation is carried out under a pressure of 15 to 25 x 10⁵ Pa.

8. Process according to one of claims 1 to 7, **characterised in that** the asymmetric hydrogenation is carried out in a protic solvent.

9. Process according to claim 8, **characterised in that** the asymmetric hydrogenation is carried out in methanol, ethanol, propanol or isopropanol as solvent.

10. Process according to claim 9, **characterised in that** the asymmetric hydrogenation is carried out in methanol as solvent.

11. Process according to one of the preceding claims 9 or 10, **characterised in that** the solvent for the asymmetric hydrogenation contains water.

12. Process according to one of the preceding claims 1 to 11, **characterised in that** the molar ratio of educt to the rhodium catalyst in the asymmetric hydrogenation is between 500:1 and 10000:1, preferably between 500:1 and 3000:1.

13. Process according to claim 12, **characterised in that** the molar ratio of educt to the rhodium catalyst in the asymmetric hydrogenation is between 1000:1 and 2000:1.

14. Process according to one of the preceding claims 1 to 13, **characterised in that** the rhodium catalyst for the asymmetric hydrogenation is used as a pre-prepared solution.

15. Process according to one of the preceding claims 1 to 13, **characterised in that** the rhodium catalyst for the asymmetric hydrogenation is produced *in situ*.

16. Process according to one of the preceding claims 1 to 14, **characterised in that** the reaction time for the asymmetric hydrogenation is between 2 and 8 hours, preferably between 4 and 6 hours.

17. Process according to one of the preceding claims 1 to 16, **characterised in that** in the second reaction step a nitrogen base, preferably ammonia, is used as the base for precipitating the N-protected adrenaline.

18. Process according to one of the preceding claims 1 to 17, **characterised in that** in the third reaction step sulphuric acid, hydrochloric acid or phosphoric acid, preferably sulphuric acid, is used to acidify the solvent.

19. Process according to one of the preceding claims 1 to 18, **characterised in that** the pH of the third step is between 5 and 6.

20. Process according to one of the preceding claims 1 to 19, **characterised in that** the reaction temperature of the third reaction step is 40 to 80°C, preferably 50 to 70°C and most preferably 60°C.

## Revendications

1. Procédé de fabrication d'adrénaline ou d'un sel d'addition d'adrénaline, **caractérisé en ce que**, dans une première étape, on soumet comme éduit de l'adrénalon protégé sur l'azote à une hydrogénation asymétrique avec du [Rh(COD)Cl]₂ et à un ligand phosphine chiral et bidendate comme système catalyseur, puis au cours d'une deuxième étape, l'adrénaline protégée sur l'azote qui en résulte est précipitée en milieu basique et, lors d'une troisième étape, l'azote est déprotégé en milieu acide.

2. Procédé selon la revendication 1, **caractérisé en ce qu'**on utilise dans la première étape comme éduit du N-benzyl-adrénalon **1**, qui est transformé lors de la première étape de la réaction en N-benzyl-adrénaline **2**, qui est précipitée lors de la deuxième étape, et, lors de la troisième étape, est convertie en adrénaline ou en son sel d'addition d'acide, par séparation du groupe benzylique protecteur de l'azote par hydrogénation en présence d'un catalyseur au palladium, de préférence du palladium sur charbon.

3. Procédé selon la revendication 1 ou 2, **caractérisé en ce que** le ligand de phosphine est la (2R,4R)-4-(dicyclohexylphosphino)-2-(diphénylphosphino-methyl)-N-méthyl-aminocarbonyl-pyrrolidine.

4. Procédé selon la revendication 1 ou 2, **caractérisé en ce que** le ligand de phosphine est de la (2R,4R)-4-(dicyclohexylphosphino)-2-(diphénylphosphino-methyl)-N-méthyl-aminocarbonyl-pyrrolidine liée à un polymère.

5. Procédé selon l'une quelconque des revendications 1 à 4, **caractérisé en ce que** l'hydrogénation asymétrique est effectuée dans une plage de température de 40°C à 70°C, de préférence de 45°C à 55°C.

6. Procédé selon l'une quelconque des revendications précédentes 1 à 5, **caractérisé en ce que** l'hydrogénation asymétrique est effectuée sous une pression de 10 x 105 Pa à 100 x 10⁵ Pa, de préférence 10 à 50 x 10⁵ Pa.

7. Procédé selon la revendication 6, **caractérisé en ce que** l'hydrogénation asymétrique est effectuée sous une pression de 15 à 25 x 10⁵ Pa.

8. Procédé selon l'une quelconque des revendications 1 à 7, **caractérisé en ce que** l'hydrogénation asymétrique est effectuée dans un solvant protique.

9. Procédé selon la revendication 8, **caractérisé en ce que** l'hydrogénation asymétrique est effectuée dans du méthanol, de l'éthanol, du propanol ou de l'isopropanol comme solvant.

10. Procédé selon la revendication 9, **caractérisé en ce que** l'hydrogénation asymétrique est effectuée dans du méthanol comme solvant.

11. Procédé selon l'une quelconque des revendications précédentes 9 ou 10, **caractérisé en ce que** le solvant pour l'hydrogénation asymétrique contient de l'eau.

12. Procédé selon l'une quelconque des revendications précédentes 1 à 11, **caractérisé en ce que** le rapport molaire entre l'éduit et le catalyseur au rhodium pour l'hydrogénation asymétrique se situe entre 500: 1 et 10000: 1, de préférence entre 500: 1 et 3000: 1.

13. Procédé selon la revendication 12, **caractérisé en ce que** le rapport molaire entre l'éduit et le catalyseur au rhodium dans l'hydrogénation asymétrique se situe entre 1000: 1 et 2000: 1.

14. Procédé selon l'une quelconque des revendications précédentes 1 à 13, **caractérisé en ce que** le catalyseur au rhodium pour l'hydrogénation asymétrique est utilisé comme solution préfabriquée.

15. Procédé selon l'une quelconque des revendications précédentes 1 à 13, **caractérisé en ce que** le catalyseur au rhodium pour l'hydrogénation asymétrique est fabriqué *in situ*.

16. Procédé selon l'une quelconque des revendications précédentes 1 à 14, **caractérisé en ce que** le temps de réaction pour l'hydrogénation asymétrique se situe entre 2 et 8 heures, de préférence entre 4 et 6 heures.

17. Procédé selon l'une quelconque des revendications précédentes 1 à 16, **caractérisé en ce que**, dans la deuxième étape de réaction, on utilise comme base une base azotée, de préférence de l'ammoniac, pour la précipitation de l'adrénaline protégée sur son azote.

18. Procédé selon l'une quelconque des revendications précédentes 1 à 17, **caractérisé en ce que**, lors de la troisième étape de réaction, on utilise de l'acide sulfurique, de l'acide chlorhydrique ou de l'acide phosphorique, de préférence de l'acide sulfurique pour l'acidification du solvant.

19. Procédé selon l'une quelconque des revendications précédentes 1 à 18, **caractérisé en ce que** le pH de la troisième étape se situe entre 5 et 6.

20. Procédé selon l'une quelconque des revendications précédentes 1 à 19, **caractérisé en ce que** la température de réaction de la troisième étape de réaction est de 40 à 80°C, de préférence de 50 à 70°C et avec une préférence particulière de 60°C.
